# NEW EUROPEAN PATENT SPECIFICATION

(11) **EP 1 663 380 B2**
(45) Date of publication and mention of the opposition decision: **03.07.2024**
(45) Mention of the grant of the patent: 29.02.2012
(21) Application number: 04778864.1
(22) Date of filing: 21.07.2004
(51) Int. Cl.: A61M 35/00, A61F 13/00

(54) **NEGATIVE PRESSURE WOUND TREATMENT DRESSING**
UNTERDRUCK-WUNDBEHANDLUNGSVERBAND
PANSEMENT DE TRAITEMENT DE PLAIES PAR PRESSION NEGATIVE

(30) Priority: 22.07.2003 US 489344 P
(43) Date of publication of application: 07.06.2006
(60) Divisional application: 10176295.3
(73) Proprietor: KCI Licensing, Inc., San Antonio, TX 78265 (US)
(72) Inventor: WILKES, Robert, P., San Antonio, TX 78231 (US); SANDERS, Teryl, Blane, San Antonio, TX 78258 (US)
(74) Representative: Simmons & Simmons
(86) International application number: PCT/US2004/023541
(87) International publication number: WO 2005/009488

(56) References cited:
- WO-A1-94/20041
- DE-A1- 19 844 355
- GB-A- 2 365 350
- US-A- 6 071 267
- US-A- 6 071 267
- US-A- 6 135 116
- US-A1- 2001 043 943
- US-A1- 2002 017 304
- US-A1- 2002 082 567
- US-A1- 2002 082 567
- US-A1- 2002 128 678
- US-A1- 2002 161 346
- US-A1- 2003 050 594
- US-B2- 9 220 299
- ADVANCES-IN-SKIN& WOUND CARE, Vol -16-N°6 p-276
- D. Dill-Muller A. Benowitz, A. Wagner,W. Tilgen "Vakuumassistierter Wunderschluss nach Exzision eines malignen Melanoms an der Ferse mit dem Fersenschaum (V.A.C-Heeldressing), Zen tralbl Chir 2004; 129 S108-S112 DOI 101055/S-2004-822670
- P.Mauckner "Die Behandlung des Fersendekubitus mit der Vakuurntherapie unter Verwendung des neu artigen VAC Heel-dressings" Zentralbl Chir 2004 129:S104-S107 DOI.IO 1055/S-2004-822619
- www.thieme-connect.com/ejournals/issue/10. 1055/S-00-23376

## Description

### FIELD OF THE INVENTION

This invention relates to the treatment of open, chronic wounds, as well as burns and skin grafts. More specifically this invention relates to dressings for the treatment of wounds to the extremities, such as the heel of a human foot, by means of negative pressure wound therapy.

### BACKGROUND OF THE INVENTION

Negative pressure wound therapy has been utilized for the treatment of open wounds and has been commercialized by Kinetic Concepts, Inc. of San Antonio, Texas, by its proprietary V.A.C.^{®} product line. In practice, the application to a wound of negative gauge pressure, commercialized by Assignee or its parent under the designation "Vacuum Assisted Closure^{®}" (or "V.A.C.^{®}") therapy, typically involves the mechanical-like contraction of the wound with simultaneous removal of excess fluid, which is often accomplished by means of a polymer foam and occlusive drape dressing in fluid communication with a negative pressure source. In this manner, negative pressure wound therapy augments the body's natural inflammatory process while alleviating many of the known intrinsic side effects, such as the production of edema caused by increased blood flow absent the necessary vascular structure for proper venous return. As a result, negative pressure wound therapy has been highly successful in the promotion of wound closure, healing many wounds previously thought largely untreatable.

Many of these wounds include decubitus and venous stasis ulcers to the lower extremities, especially the foot. Closure of these wounds has been difficult and often times impossible using traditional techniques, such as skin grafting, sharp debridement, or combinations thereof. Failure to close these wounds, which have often been present for several years, can lead to necrotizing of the tissue, and in many cases amputation of the extremity. Use of negative pressure wound therapy has proven highly successful in closing these wounds. However, treatment of the lower extremities with negative pressure wound therapy, especially to wounds of the foot and heal can be difficult, especially considering the nature of the location of the wound. Particular concern arises with maintaining the dressing on the extremity, especially in light of the frequent movement of the foot, and friction often associated with foot coverings, including socks, stockings, and shoes. Of particular concern, is the ability to maintain a negative pressure at the wound site when the dressing is in place, as air leaks may occur during movement of the foot, which can adversely affect the therapy being administered.

For the foregoing reasons, there is a need for a negative pressure wound treatment dressing system that is capable of maintaining a negative pressure over an extremity that has heretofore been difficult to treat using traditional negative pressure wound therapy dressings. Additionally, there is a need for a negative pressure wound treatment dressing system that is capable of maintaining a negative pressure over a wound which exists in a highly contoured part of the body, or a portion of the body that undergoes frequent movement or friction against clothing or other outside forces.

It is therefore an object of the present invention to provide a negative pressure wound treatment dressing system that provides a means for maintaining a negative pressure over a contoured extremity, such as the foot, and more specifically the heel of the foot.
US 2001/0043943 A1 discloses a wound care bandage for treating a wound, including an SIS layer to be placed on the wound, a cover to be placed over the wound and a structure to provide a vacuum space.
US 2002/0082567 A1 discloses a thin, flexible member for use in a vacuum bandage connected to a vacuum source, for use with a wound having a wound surface. The member includes a wound contacting surface adapted to be in contact with and generally conform to the wound surface. The member further includes a plurality of discrete holes formed in the wound contacting surface, a port configured to communicate with the vacuum source, and communicating means from the holes to the port. The member is formed from a generally non-porous material.
US 6,135,116 discloses a method and apparatus for providing concurrent applications of intermittent pneumatic compression therapy and vacuum assited closure therapy, comprising a wound dressing for introduction of a negative pressure into a wound, and a foot wrap for application of positive, compressive forces.
US 2002/0017304 A1 discloses a wound therapy combination comprising a suction head and a surgical drape. The suction head comprises a planar flange portion and a tubular connector piece on a first face that communicates with an aperture extending to a second face. The second face is formed with projections that define flow channels for facilitating flow of liquids to the aperture. US 6,071,267 discloses a patient interface system including a fluid transfer subsystem comprising a primary fluid transfer element fluidically communicating with the wound and a secondary fluid transfer element in contact therewith. A drape subsystem covers the fluid transfer elements and includes a film material membrane. A fluid conveying subsystem includes a vacuum source connected to the secondary fluid transfer element by a suction tube for creating a negative pressure for extracting fluids. A fluid supply system is provided for supplying various types of fluids to the interface system.
DE 198 44355 A1 discloses an adhesive wound dressing having a flexible membrane, and an integrated device for production of a negative pressure for suction.

### SUMMARY

In a first aspect of the invention there is provided a wound dressing as defined in claim 1 of the appended claims.

In accordance with the foregoing objects, in embodiments the present invention generally comprises a contoured porous pad positioned within a fenestrated and contoured occlusive wrapping for placement over a wound on an extremity, such as the heel of a human foot. A concave porous pad is placed adjacent or within the wound, such that the concave pad is enveloped by the occlusive wrapping. Flexible tubing is attached to or through an opening of the drape, so as to allow for fluid communication of negative pressure to the concave pad from a source of negative pressure connected to an opposite end of the flexible tubing. The negative gauge pressure is communicated from the source, through the tube, through the fenestrations of the occlusive wrapping, such that negative gauge pressure is applied to the wound on the extremity. The contoured porous pad serves to manifold the negative pressure from a position away from the wound site to the concave pad at the wound site. Such an arrangement allows for the tubing to connect to the dressing of the present invention at a position least likely to be dislodged during normal ambulation of the patient.

Attachments means are formed on the outer edges of the occlusive wrapping to form an airtight seal over the wound. Such attachment means may be an adhesive coated on the occlusive wrapping for attachment to an intact area of tissue or skin. The adhesive is coated on the perimeter edge of the occlusive wrapping such that a pouch is formed when the occlusive wrapping is secured over the concave pad.

A collection canister is provided between the negative pressure source and the occlusive drape for collecting any effluents that may be drawn from the wound during application of negative pressure.

The tubing is connected to the drape and pad through an opening in the drape, or through a flanged tubing connector as has been described in U. S. Patent 6,345, 623, entitled "Surgical drape and suction head for wound treatment," to Heaton, et al. Alternative embodiments allow the tubing to be placed within or embedded in the concave padding.

The foregoing has outlined some of the more pertinent objects of the present invention. These objects should be construed to be merely illustrative of some of the more prominent features and applications of the invention. Many other beneficial results can be attained by applying the disclosed invention in a different manner or by modifying the invention as will be described. Accordingly, other objects and a fuller understanding of the invention may be had by referring to the following Detailed Description of the Invention, which includes the preferred embodiment.

### BRIEF DESCRIPTION OF THE DRAWINGS

These and other features and advantages of the invention will now be described with reference to the drawings of certain preferred embodiments, which are intended to illustrate and not to limit the invention, and wherein like reference numbers refer to like components, and in which:
Figure 1A, is an exploded view of the occlusive wrapping of the present invention.
Figure 1B, is an orthogonal flat pattern view of the occlusive wrapping of the present invention.
Figure 2 is an orthogonal view of the fluid manifold of the occlusive wrapping of the present invention.
Figure 3A is a perspective view of a contoured pad of the present invention.
Figures 3B and 3C are cross-sectional views of a contoured pad of the present invention.
Figure 3D is a top plane view of the contoured pad of the present invention.
Figures 4A-4G are perspective views of the negative pressure wound treatment dressing system, illustrating the steps of applying the dressing system to a wound on the heel of a foot.
Figure 5 is an orthogonal flat pattern view of the occlusive wrapping of the present invention folded along its center line.

### DETAILED DESCRIPTION

Although those of ordinary skill in the art will readily recognize many alternative embodiments, especially in light of the illustrations provided herein, this detailed description is exemplary of the preferred embodiment of the present invention as well as alternate embodiments, the scope of which is limited only by the claims that may be drawn hereto.

The details of the preferred embodiments of present invention are graphically and schematically illustrated in the accompanying drawings. Like elements in the drawings are represented by like numbers, and any similar elements are represented by like numbers with a different lower case suffix.

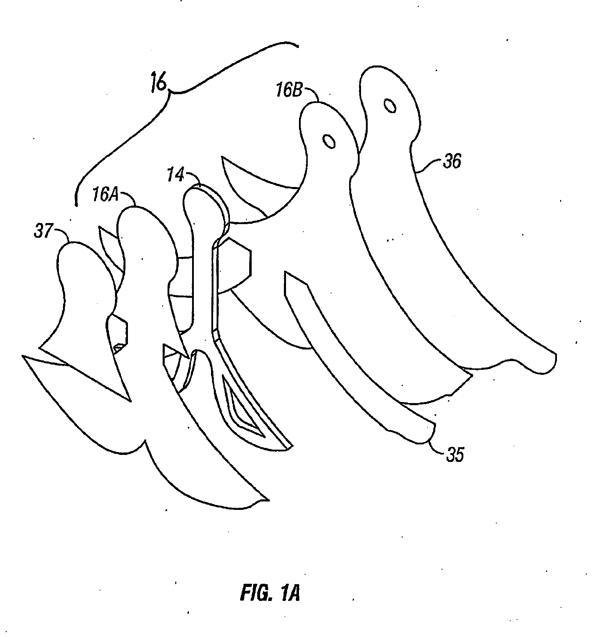

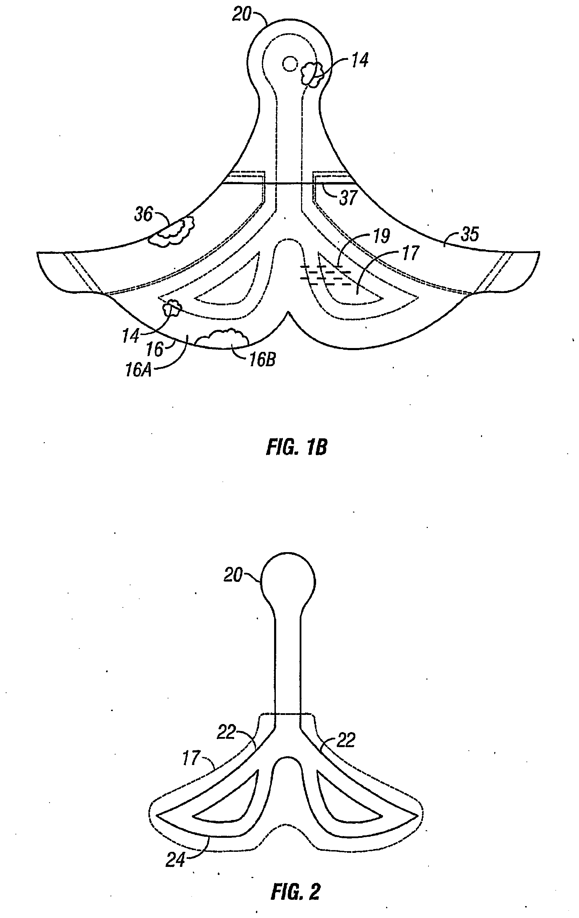

Referring now to Figure 1 in particular, there is illustrated the primary components of the occlusive wrapping **10** of the negative pressure dressing system that operates in accordance with the present invention. The occlusive wrapping **10** consists of a fluid manifold **14** embedded within an occlusive drape **16.** The occlusive drape **16** is preferably made of a clear vapor permeable polyurethane material. The drape **16** is composed of a wound facing layer **16a** and an outer layer **16b** that are sealed along their periphery to secure the fluid manifold **14** within the wound facing layer **16a** and the outer layer **16b** of the drape **16.** Multiple fenestrations **19** are formed along a wound contact region **17** of the wound facing layer **16a,** so as to allow for fluid communication through the fluid manifold **14.** The fluid manifold **14** allows for attachment of the fluid communication port **18** at a position away from the wound site. The type of wounds desired to be treated by the present system are often situated at a position that has, at times been difficult to maintain a reduced pressure using traditional negative pressure wound therapy techniques described herein. The occlusive wrapping **10** of the present invention overcomes this difficulty by communicating the negative pressure along the manifold **14,** and more particularly the fluid manifold **14** of the occlusive wrapping **10,** which is most clearly illustrated in Figure 2.

Figure 2 illustrates an alternative embodiment of the fluid manifold **14** of the occlusive wrapping **10.** The fluid communication port **18** is attached to a receiving site **20,** which is most preferably a circular shaped foam section that is approximately the size of the fluid communication port **18.** Fluid communicator arms **22** extend distally from the receiving site **20** to the wound contact regions **17.** The fluid communicator arms 22 terminate in loops 24, having openings for viewing the wound perimeter. In an alternative embodiment, fluid communicator fingers **24** extend distally from the fluid communicator arms **22.**

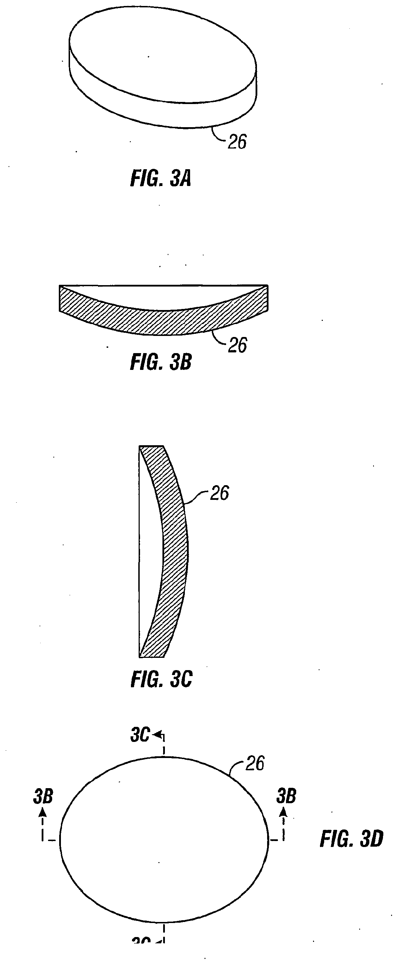

A contoured pad 26 as illustrated in Figures 3A-3D is utilized as a wound contact screen to prevent unwanted adhesions, direct fluid away from the wound through pores within the pad 26, and exert contracting forces on the wound margins as negative pressure is applied through the manifold 14 and to the pad 26. The contoured pad 26 and fluid manifold 14 of the occlusive wrapping 10 are made of a porous polymer foam material, such as polyurethane or polyvinyl alcohol foam. The pad 26 is preferably constructed into an elliptical shape as illustrated in Figure 3D, but may also be more symmetrically circular to accommodate more uniform wounds.

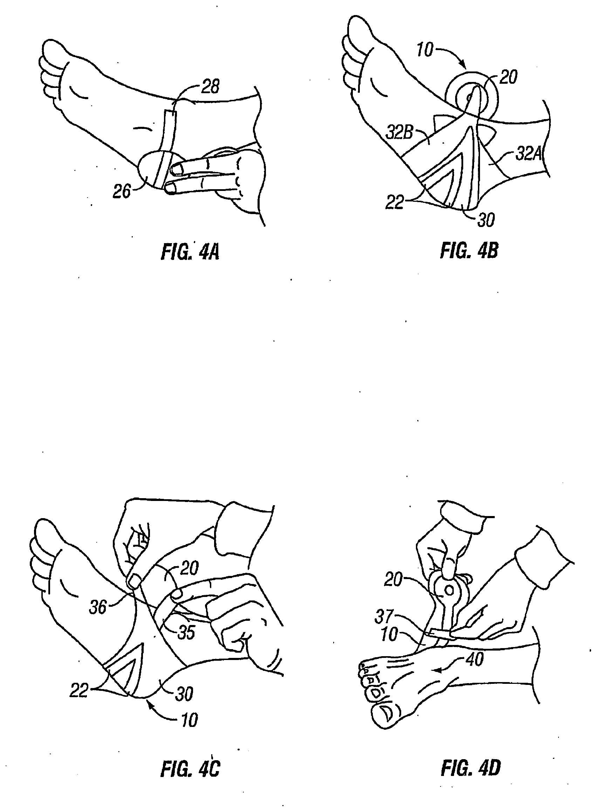

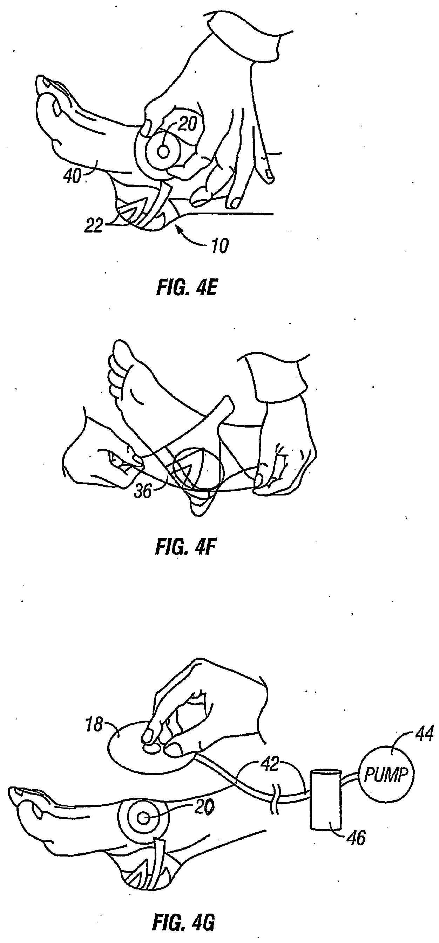

In practice, the contoured pad 26 is trimmed to the areal dimensions of the wound and is placed in the wound, which may be on the heel of a foot, and between the wound and the fluid manifold 14, as illustrated in Figures 4A-4G. The pad 26 may be secured to the wound site by an adhesive strip 28, as shown in Figure 4A, or by other means known in the art. In the preferred embodiment, the adhesive strip is constructed of the same vapor permeable polyurethane material as the occlusive drape 16, having an adhesive coated on one side for securing the strip to the pad 26 and the intact skin surrounding the wound.

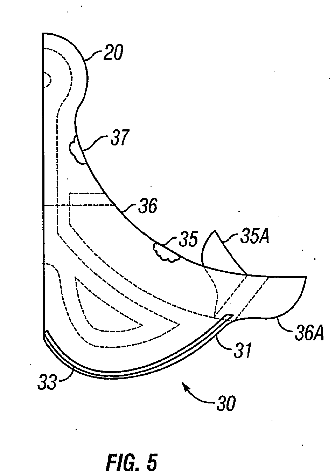

The occlusive wrapping 10 forms a pouch 30 when it is folded along its centerline and the lower edges 31 are bonded together at 33 as shown in Figure 5. In practice, the pouch 30 is placed over the wound and contoured pad 26, as illustrated in Figures 4B and 4C. A fingerhold 36a is provided to facilitate grasping the pouch 30 and holding it in place as the liner 35 is removed to expose an adhesive backing on layer 16b. The edges 32a, 32b of the pouch 30 are brought into contact with the intact skin surrounding the wound by means of an adhesive backing. A removable liner**35** is pulled off, starting at finger tab **35a**, exposing the adhesive, as shown in Figure 4C. The removable liner **35** may be statically adhered to the adhesive backing to protect the adhesive during handling.

As illustrated in Figure 4D, a second removable liner **37** is adhered to the receiving site **20** to protect the adhesive on the wound facing layer **16a** along the receiving site **20** during handling, and which is removed prior to adhesion of the receiving site **20** to a region **40** of intact skin away from the wound site, so as to prevent dislodgement of the fluid communication port **18** during movement of the patient, as shown in Figure 4E. A removable, protective liner **36** may be overlaid on the occlusive drape **16,** so as to provide rigidity to the manifold during handling, and which is removed after securing the pouch **30** to the wound site, as shown in Figure 4F.

A fluid communication port **18** is positioned over the receiving site **20,** and is secured in place also by means of an adhesive contact surface on the communication port **18.** As known in the art of negative pressure wound therapy, a fluid communication means **42,** such as a flexible silicone tube, communicates negative pressure to the wound from a negative pressure source **44,** such as a portable pump. A collection canister **46** is positioned in line between the negative pressure source **44** and the fluid communication means **42** to collect any exudates that may be drawn from the wound.

While the invention has been described herein with reference to certain preferred embodiments, theses embodiments have been presented by way of example only, and not to limit the scope of the invention. Many other variations are possible, which would be obvious to one skilled in the art. Accordingly, the scope of the invention should be identified only in accordance with the claims that follow.

## Claims

1. A wound dressing for use under negative pressure, comprising:
a porous polymer foam concave pad adapted for placement in a wound on an extremity;
an occlusive wrapping adapted for positioning over the porous polymer foam concave pad, wherein said occlusive wrapping comprises a porous polymer foam fluid manifold having a receiving site and fluid communicator arms extending distally from the receiving site, and an occlusive drape which is made of a vapor permeable polyurethane material, surrounding said porous polymer foam fluid manifold, wherein the occlusive drape has a wound facing layer and an outer layer and wherein a wound contact region of the wound facing layer is fenestrated, and an adhesive on at least an edge of said occlusive drape for sealing said occlusive wrapping to the area surrounding the wound; and
a fluid communication port through at least a layer of said occlusive drape for communicating fluid from said porous polymer foam fluid manifold to a source of negative pressure,
wherein the occlusive wrapping is folded along its centreline and its lower edges are bonded together to form a pouch for receiving at least a portion of the extremity.

2. The wound dressing of claim 1, wherein said communicator arms (22) form contiguous loops (24).

3. A wound dressing for use under negative pressure according to claim 1 wherein the occlusive drape (16) is clear, and wherein the fluid communicator arms (22) terminate in contiguous loops (24) to allow viewing of the area around the wound.

4. The wound dressing of claim 3, wherein said portion of the drape that is fenestrated is on said wound facing layer (16A).

5. A wound dressing for use under negative pressure according to claim 2 wherein the occlusive drape (16) is clear to allow viewing of the area around the wound through the occlusive drape (16).

## Patentansprüche

1. Ein Wundverband zur Verwendung unter Unterdruck, umfassend:
ein konkaves Polster aus porösem Polymerschaum, das zur Platzierung in einer Wunde an einer Extremität geeignet ist;
eine okklusive Umhüllung, die zum Positionieren über dem konkaven Polster aus porösem Polymerschaum geeignet ist, wobei die okklusive Umhüllung einen Fluidverteiler aus porösem Polymerschaum mit einer Aufnahmestelle und Fluidverbindungsarmen, die sich distal von der Aufnahmestelle erstrecken, und ein okklusives Abdecktuch, das aus einem dampfdurchlässigen Polyurethanmaterial hergestellt ist, das den Fluidverteiler aus porösem Polymerschaum umgibt, wobei das okklusive Abdecktuch eine der Wunde zugewandte Schicht und eine äußere Schicht aufweist und wobei ein Wundkontaktbereich der der Wunde zugewandten Schicht fenestriert ist, und einen Klebstoff an mindestens einem Rand des okklusiven Abdecktuchs zum Versiegeln der okklusiven Umhüllung zu dem die Wunde umgebenden Bereich umfasst; und
eine Fluidverbindungsöffnung durch mindestens eine Schicht des okklusiven Abdecktuchs, um Fluid aus dem Fluidverteiler aus porösem Polymerschaum zu einer Unterdruckquelle zu leiten,
wobei die okklusive Umhüllung entlang ihrer Mittellinie gefaltet ist und ihre unteren Kanten miteinander verbunden sind, um einen Beutel zum Aufnehmen mindestens eines Abschnitts der Extremität zu bilden.

2. Der Wundverband nach Anspruch 1, wobei die Verbindungsarme (22) zusammenhängende Schlaufen (24) bilden.

3. Ein Wundverband zur Verwendung unter Unterdruck nach Anspruch 1, wobei das okklusive Abdecktuch (16) durchsichtig ist und wobei die Fluidverbindungsarme (22) in zusammenhängenden Schlaufen (24) enden, um die Sicht auf den Bereich um die Wunde herum zu ermöglichen.

4. Der Wundverband nach Anspruch 3, wobei der Abschnitt des Abdecktuchs, der fenestriert ist, auf der der Wunde zugewandten Schicht (16A) ist.

5. Ein Wundverband zur Verwendung unter Unterdruck nach Anspruch 2, wobei das okklusive Abdecktuch (16) durchsichtig ist, um die Sicht auf den Bereich um die Wunde herum durch das okklusive Abdecktuch (16) zu ermöglichen.

## Revendications

1. Pansement pour plaie destiné à être utilisé sous pression négative, comprenant :
un tampon concave de mousse polymère poreux conçu pour être placé dans une plaie sur une extrémité ;
une enveloppe occlusive conçue pour positionner sur le tampon concave de mousse polymère poreux, dans lequel ladite enveloppe occlusive comprend un collecteur de fluide en mousse polymère poreux ayant un site de réception et des bras de communication fluidique s'étendant de manière distale à partir du site de réception, et un champ occlusif qui est constitué d'un matériau polyuréthane perméable à la vapeur, entourant ledit collecteur de fluide en mousse polymère poreux, dans lequel la protection occlusive a une couche faisant face à la plaie et une couche externe et dans lequel une région de contact avec la plaie de la couche faisant face à la plaie est coupée, et un adhésif sur au moins un bord dudit champ occlusif pour sceller ladite enveloppe occlusive vers la zone entourant la plaie ; et
un orifice de communication de fluide à travers au moins une couche de champ occlusif pour communiquer un fluide à partir dudit collecteur de fluide en mousse polymère poreux à une source de pression négative,
dans lequel l'enveloppe occlusive est pliée le long de sa ligne centrale et ses bords inférieurs sont liés ensemble pour former une poche pour recevoir au moins une partie de l'extrémité.

2. Pansement pour plaie selon la revendication 1, dans lequel lesdits bras de communication (22) forment des boucles contiguës (24).

3. Pansement pour plaie destiné à être utilisé sous pression négative selon la revendication 1, dans lequel le champ occlusif (16) est dégagé, et dans lequel les bras de communication de fluide (22) se terminent dans des boucles contiguës (24) pour permettre la visualisation de la zone autour de la plaie.

4. Pansement pour plaie selon la revendication 3, dans lequel ladite partie du champ qui est coupée est sur ladite couche faisant face à la plaie (16A).

5. Pansement pour plaie destiné à être utilisé sous pression négative selon la revendication 2, dans lequel le champ occlusif (16) est dégagé pour permettre la visualisation de la zone autour de la plaie à travers le champ occlusif (16).
